# EUROPEAN PATENT SPECIFICATION

(11) **EP 4 241 674 B1**
(45) Date of publication and mention of the grant of the patent: **27.05.2026**
(21) Application number: 22161739.2
(22) Date of filing: 11.03.2022
(51) Int. Cl.: A61B 5/08, A61B 5/00, A61B 5/11, A61B 5/0538

(54) **METHOD AND SYSTEM FOR MEASURING A DEGREE OF MUSCLE DISPLACEMENT AND USE OF THE METHOD AND SYSTEM IN TREATING OBSTRUCTIVE SLEEP APNEA**
VERFAHREN UND SYSTEM ZUR MESSUNG EINES MUSKELVERSCHIEBUNGSGRADES UND VERWENDUNG DES VERFAHRENS UND SYSTEMS ZUR BEHANDLUNG VON OBSTRUKTIVER SCHLAFAPNOE
PROCÉDÉ ET SYSTÈME DE MESURE D'UN DEGRÉ DE DÉPLACEMENT MUSCULAIRE ET UTILISATION DU PROCÉDÉ ET DU SYSTÈME DANS LE TRAITEMENT DE L'APNÉE OBSTRUCTIVE DU SOMMEIL

(43) Date of publication of application: 13.09.2023
(73) Proprietor: Nyxoah SA, 1435 Mont-St-Guibert (BE)
(72) Inventor: Kohn, Sarah, Modiin 7174661 (IL); Tsukran. Roi, Rishon Le-Zion 7575721 (IL)
(74) Representative: Brantsandpatents bv

(56) References cited:
- WO-A1-2021/242633
- US-A1- 2013 204 314
- US-A1- 2014 135 868
- US-A1- 2015 224 307
- US-A1- 2018 221 657

## Description

The invention described herein refers to a system according to claim 1.

Neural modulation, i.e. electrical stimulation of nerves, is well-known in the prior art as a reliable and effective type of medical treatment. It presents the opportunity to tackle many physiological conditions and disorders by interacting with the body's own natural neural processes. Neural modulation includes inhibition (e.g. blockage), stimulation, modification, regulation, or therapeutic alteration of activity, electrical or chemical, in the central, peripheral, or autonomic nervous system. By modulating the activity of the nervous system, several different goals may be achieved. For instance, motor neurons may be stimulated at appropriate times to cause muscle contractions. Further, sensory neurons can be blocked to relieve pain or stimulated to provide a signal to a subject. In yet other examples, modulation of the autonomy nervous system may be used to adjust various involuntary physiological parameters, such as heart rate and blood pressure. Neural modulation may provide the opportunity to treat several diseases or physiological conditions. Various devices and techniques have been used in attempts to provide optimum stimulation of a tissue of interest.

Within the content of this disclosure, the expressions "nerve stimulation", "neural stimulation", "nerve modulation" and "neural modulation" are used synonymously unless something else is apparent from the respective context. In general, the above expressions refer to the process of generating an electric field in the vicinity of a nerve or a group of nerves in order to cause contraction of one or more muscles associated to said nerves. Likewise, the expressions "subject" and "patient" are used synonymously unless something else is apparent from the respective context. Both terms refer to a person potentially suffering from OSA.

One of the conditions to which neural modulation can be applied to is obstructive sleep apnea (OSA), a respiratory disorder characterized by recurrent episodes of partial or complete obstruction of the upper airway during sleep. One of the major causes of OSA is the inability of the tongue muscles to resist negative inspiratory pressure in the pharynx due to the sleep-related loss in muscle tone. As the tongue is pulled backwards, it obstructs the upper airway, decreasing ventilation and lowering lung and blood oxygen levels. Stimulation of the hypoglossal nerve ("Hypoglossal Nerve Stimulation" or HGNS) causes the tongue muscles to contract, thereby maintaining an open, unobstructed airway. When a person without OSA is sleeping, the pharyngeal muscles - a group of muscles that form the pharynx - relax and gradually collapse, narrowing the airway. Narrowing of the airway in turn limits the effectiveness of the sleeper's breathing, causing a rise in CO₂ levels in the blood of the sleeper. The increase in CO₂ results in the pharyngeal muscles contracting to open the airway to restore proper breathing. The larger of the pharyngeal muscles responsible for upper airway dilation is the genioglossus muscle, which is one of several different muscles in the tongue.

The genioglossus muscle is responsible for the forward movement of the tongue as well as for the stiffening of the anterior pharyngeal wall. In patients with OSA, the neuromuscular activity of the genioglossus muscle is decreased compared to normal individuals, accounting for insufficient response and contraction to open the airway as compared to a normal individual. This lack of response contributes to a partial or total airway obstruction, which significantly limits the effectiveness of the sleeper's breathing. In OSA patients, there are often several airway obstruction events during the night. Because of the obstruction, there is a gradual decrease of O₂ levels in the blood (hypoxemia). Hypoxemia leads to night time arousals, which may be registered by EEG, showing that the brain awakes from any stage of sleep to a short arousal. During the arousal, there is a conscious breath or gasp, which resolves the airway obstruction. An increase in sympathetic tone activity rate through the release of hormones such as epinephrine and noradrenaline also often occurs as a response to hypoxemia. Because of the increase in sympathetic tone, the heart enlarges in an attempt to pump more blood and increase the blood pressure and heart rate, further arousing the patient after the resolution of the apnea event, as the patient returns to sleep, the airway collapses again, leading to further arousals.

In order to quantify the efficacy of neural modulation as a therapeutic method (in particular HGNS) as well as for analytical purposes, it is important that therapy response is closely monitored. This is usually achieved via through monitoring of nasal and/or oral airflow of the subject, e.g. with the use of PSG (sleep study or Polysomnography) or CPAP (Continuous Positive Airway Pressure) masks. Alternatively, patients can self-assess therapy efficacy during wakeful titration. Other methods may include visually observing airway opening or tongue protrusion to assess if the therapy resulted in the desired motor response. Further assessments of therapy response include drug-induced or wakeful endoscopy.

WO 2021/242633 A1 describes devices and methods for treating sleep disordered breathing.

US 2018/0221657 A1 describes a method for delivering energy as a function of degree coupling which utilizes an external unit configured for location external to a body of a subject.

US 2015/0224307 A1 describes a method of providing a sleep apnea nerve stimulation therapy to a subject.

US 2014/0135868 A1 describes a non-invasive removable intraoral electrical stimulator or pacemaker system.

US 2013/0204314 A1 describes methods and systems for determining appropriate situations to treat conditions such as sleep apnea.

The above methods have several disadvantages. Solutions involving airflow are not suitable for seamless implementation in existing systems for neuro stimulation. Furthermore, using masks and the like may be perceived as annoying by the subject. As for self-assessment or visualization of airway opening or tongue protrusion, these methods can be highly subjective and are therefore subject to variating results or mistakes. Furthermore, even objective visual assessments merely provide qualitative results rather than quantitative.

The objective technical problem of the present disclosure is to eliminate the disadvantages of the prior art and to provide a method for measuring a degree of airway opening, e.g. of muscle displacement in order to facilitate quantification of the efficacy of neural modulation therapy. Furthermore, the method should improve electrical nerve stimulation in a patient associated with OSA.

### Summary of the invention

Main features of the method according to the disclosure are specified by claim 1.

According to an example that is not covered by the present invention, a method for measuring a degree of muscle collapse and/or contraction of a subject in response to electrical nerve stimulation is described, the method comprising:
- generating a modulation signal, the modulation signal comprising an electrical stimulation pattern;
- applying the modulation signal to at least one pair of electrodes associated with an implant unit implanted inside the subject's body;
- detecting a movement of one or more muscles related to the subject's airway in response to the modulation signal applied to the at least one pair of electrodes;
- assigning to the determined movement a value that is indicative of the degree of airway opening and/or obstruction, e.g. of muscle displacement.

With the method as described above, the efficacy of nerve stimulation during treatment of patients suffering from OSA, in particular the efficacy of stimulation of the hypoglossal nerve or of the ansa cervicalis, can be monitored in an easy and accurate manner. It is understood that the hypoglossal nerve innervates the genioglossus muscle and that the ansa cervicalis innervates the infrahyoid strap muscles, which are responsible for stiffening or collapsing the upper airway of a subject. The infrahyoid muscles include the sternohyoid muscle, the sternothyroid muscle, the omohyoid muscle, and the thyrohyoid muscle. Furthermore, if needed, the system allows for continuous monitoring over a desired time period, providing important information regarding the long-term effects of nerve stimulation therapy.

Muscle contraction in the sense of this disclosure may refer to muscle displacement, and these expressions are used synonymously unless indicated otherwise. Muscle contraction may in particular refer to (but is not limited to) displacement of the subject's tongue or of the other muscles associated to the subject's airway.

Movement of one or more muscles related to the subject's airway may, in the sense of this disclosure, also comprise movement of the chin of the subject, since contraction of one or more of the muscles involved in tongue movement will also result in the chin being moved. Thus, the degree of airway opening and/or of airway obstruction, e.g. of muscle displacement may also be determined by measurement of the subject's chin movement.

The modulation signal comprising the electrical stimulation pattern is generated by an external device, which is configured for placement underneath the subject's chin. In particular, the modulation signal may be generated by a control unit and/or by a processor, which can both be part of the external unit. The modulation signal may further be transmitted to an implant unit using a transmitting element, which may comprise an antenna or a coil associated with the control unit.

According to another example not covered by the present invention, the control unit may be part of the implant unit implanted beneath the subject's skin, either instead of or in addition to a control unit of the external device. This way, the control unit may be located directly on the muscle to be monitored, e.g. on the genioglossus muscle or on one or more of the infrahyoid muscles, in particular on the sternohyiod or the sternothyroid muscle. In other words, location of the control unit it is not essential for the underlying method of this disclosure. Either way, the control unit is in electrical commutation with the at least one motion sensing unit.

The step of applying the modulation signal to at least one pair of electrodes **is** implemented by the implant unit. The implant unit is implantable in a vicinity or proximity of the genioglossus muscle, which is innervated by the hypoglossal nerve. The implant unit may comprise an electric circuit which receives the electrical stimulation pattern through an electrical communication between the external device and the implant unit and in turn generates an electric field via at the least one pair of electrodes associated with the implant unit.

The step of determining a movement of one or more muscles related to the subject's airway opening in response to the modulation signal is implemented by a motion sensing unit. Likewise, the muscles related to the subject's airway opening are also responsible for airway obstruction, which can be detected using the method described herein. The method may further be characterized in that the at least one motion sensing unit comprises at least one inertial measurement unit (IMU) and/or at least one strain gauge. According to an exemplary embodiment, movement of one or more muscles related to the subject's airway opening and/or obstruction may also be determined by detecting movement of the chin of the subject, since movement of the muscles related in the subject's airway opening and/or airway obstruction and in particular a movement of a subject's tongue will also result in movement of a subject's chin.

Using the presented method, the actual physiological effect to be achieved via stimulation therapy, i.e. a contraction (or non-contraction) of the targeted muscle or group of muscles, can be monitored rather than the symptoms of the illness or disorder. As the latter may not immediately be improved by the therapy in some cases, they may be misleading when monitored and used as basis for adjusting the stimulation. For example, if a patient or subject suffers from a disorder like central sleep apnea (CSA), a measurement of the airflow in response to nerve stimulation therapy will indicate an apnea event. This would suggest non-effectiveness of the therapy and potentially lead to the decision to increase stimulation intensity. However, since stimulation therapy will not be able to prevent an apnea event in CSA in the first place, increasing or decreasing the stimulation intensity (or adjusting any other of the stimulation parameters) will not affect the condition of the patient with regards to the sleep apnea disorder. Therefore, airflow is no viable indicator for determining efficacy of nerve stimulation.

With the method described herein, the physiological condition that is directly affected by the underlying nerve stimulation therapy, i.e. muscle contraction, is monitored. This way, it is possible to obtain accurate information regarding therapy efficacy. Based on the determined degree of muscle displacement in response to stimulation with a defined set of stimulation parameters, it is further possible to adjust those parameters in order to improve therapy efficacy. For example, if a set group of stimulation parameters is not sufficient to cause tongue protrusion and open the airway, the underlying stimulation parameters may be adjusted accordingly, e.g. by increasing stimulation intensity.

According to another example not covered by the present invention, a threshold degree of airway opening and/or airway obstruction (e.g. of muscle displacement) may be pre-defined. In such a case, a value indicative of airway opening will only be assigned to the determined muscle movement if the threshold degree of airway opening is surpassed. Airway opening may for example comprise tongue protrusion caused by innervation of the genioglossus muscle, or widening of the upper airway caused by innervation of one or more of the infrahyiod muslces.

According to another example not covered by the present invention, the method further comprises the step: adjusting at least one parameter of the electrical stimulation pattern in response to the value indicative of the degree of airway opening, e.g. of muscle displacement. This way, feedback-based or closed loop stimulation method is provided, wherein the efficacy of an ongoing stimulation session is determined by measuring the degree of displacement of the subject's muscle in response to the associated nerve stimulation and then adjusting the stimulation parameters in response to the measured degree of airway opening and/or airway obstruction. The degree of airway opening and/or airway obstruction can for example be determined by detecting movement of the muscles associated with the tongue (i.e. collapse or protrusion) or by detecting movement of the subject's chin. Adjustment of the at least one stimulation parameter can occur automatically or by hand (e.g. through a physician or through the subject themselves) or both. In case of automatic adjustment, the at least one stimulation parameter can be changed after a set time delay or more or less instantly (i.e. in real-time). This process may be automatically repeated in an iterative manner until one of the automatic adjustments of the stimulation parameters results in determination of a value that is indicative of muscular contraction associated with a desired degree of airway opening and/or airway obstruction.

In order to achieve a feedback-based or closed loop stimulation session, the control unit may be configured to perform logical operations such as comparing the detected degree of movement (or non-movement) of one of the muscles related to the subject's airway to a pre-defined value and adjust one or more of the stimulation parameters accordingly. For example, in case the detected degree of tongue displacement or chin movement is below the pre-determined value, the control unit may be configured to increase stimulation intensity. Another example is, in case the degree of tongue collapse is above a pre-determined value, i.e. resulting in an obstruction of the subject's airway, the control unit may be configured to induce stimulation.

Advantageously, the movement of the muscles related to the subject's airway may comprise a muscle contraction, a change of the tongue's position and/or a change of the tongue's spatial orientation within the subject, and/or movement of the subject's chin. In particular but not exclusively, muscle contraction may refer to contraction of the genioglossus muscle, especially when innervated by the hypoglossal nerve.

It is also possible that the movement of the one or more muscles related to the subject's tongue is determined using at least one motion sensing unit. The method may further be characterized in that the at least one motion sensing unit comprises at least one inertial measurement unit and/or at least one strain gauge. According to another embodiment, the inertial measurement unit may comprise at least one accelerometer and/or at least one gyroscope and/or at least one magnetometer. Preferably, at least one accelerometer may be implemented, wherein the accelerometer may comprise a single axis accelerometer or a multiple axis accelerometer.

The at least one parameter may comprise a pulse train amplitude, a pulse train length, a single pulse frequency, a single pulse duration, a hold duration, a pulse train interval, a duty cycle, a delay time, a ramp duration, a step-down amplitude, a ramp at train onset duration, and/or a confirmatory pulse/train amplitude. The stimulation parameters may be adjusted automatically based on the value assigned to the measured degree of airway opening and/or airway obstruction, or the parameters can be adjusted by a physician or by the patients themselves upon receiving information that the current stimulation parameters are not sufficient.

According to this invention, a system for measuring a degree of airway opening and/or airway obstruction, e.g. of muscle displacement of a subject is presented, the system comprising:
- an implant unit configured for implantation in the vicinity or proximity of any muscle associated with the subject's airway, e.g. the genioglossus muscle or one of the infrahyoid strap muscles, the implant unit comprising at least one pair of electrodes;
- an external device configured for communication with the implant unit;
- at least one motion sensing unit for determining movement of one or more muscles related to the subject's airway in response to a modulation signal applied to the at least one pair of electrodes.

The system may preferably be configured for implementing the method described herein.

In addition to the above, the system comprises a control unit. The control unit may be part of the external device. In that case, the control unit may transmit modulation or stimulation input signals, e.g. the stimulation parameters, and power to the implant unit. The control unit further receives the determined data corresponding to the movement of one or more muscles related to the subject's tongue determined by the motion sensing unit, and assigns a value to the degree of airway opening and/or airway obstruction. According to a preferred embodiment, the control unit is also able to adjust at least one of the stimulation parameters in response to the value indicative of the degree of airway opening and/or airway obstruction, e.g. of muscle displacement.

Accordingly, the control unit may comprise one or more processors, which may include any electric circuit that configured to perform a logic operation on at least one input variable. The at least one processor of the control unit may therefore include one or more integrated circuits, microchips, microcontrollers, and microprocessors, which may be all or part of a central processing unit (CPU), a digital signal processor (DSP), a field programmable gate array (FPGA), or any other circuit known to those skilled in the art that may be suitable for executing instructions or performing logic operations.

The implant unit may be configured for implantation in a location that permits it to modulate a nerve (e.g. the hypoglossal nerve). The implant unit may be located in a subject such that intervening tissue exists between the implant unit and the nerve to be modulated. Intervening tissue may include muscle tissue, connective tissue, organ tissue, or any other type of biological tissue. Thus, location of the implant unit does not require immediate contact with the nerve for effective neuromodulation. The implant unit may also be located directly adjacent to nerve, such that no intervening tissue exists.

In treating OSA, the implant unit may be located on the genioglossus muscle of the patient or subject. Such a location is suitable for modulation of the hypoglossal nerve, branches of which run inside and innervate the genioglossus muscle. However, the implant unit may also be configured for placement in other locations.

The external device may be configured for location external to a patient, either directly contacting the skin or close to the skin of the patient. The external device may further be configured to be affixed to the patient, for example, by adhering to the skin of the patient, or through a band or other device configured to hold the external device in place. Adherence to the skin of the external unit may occur such that it is in the vicinity or in the proximity of the location of the implant unit. The external device may be configured for fixation to the patient. For example, the external device may be configured for placement underneath the subject's chin and/or on the front of patient's neck. The suitability of placement locations may be determined by communication between external device and implant unit. The external device may comprise a housing, wherein the housing may be any suitable container configured for retaining electrical components. In addition, the housing may be any suitable size and/or shape and may be rigid or flexible. Examples of housings for the external device may include one or more of patches, buttons, or other receptacles having varying shapes and dimensions and constructed of any suitable material. The external device may be configured to adhere to a desired location. Accordingly, in some embodiments, at least one side of the housing may include an adhesive material. The adhesive material may include a biocompatible material and may allow for a patient to adhere the external unit to the desired location and remove the external device upon completion of use. The adhesive may be configured for single or multiple uses of the external unit. Suitable adhesive materials may include, but are not limited to biocompatible glues, starches, elastomers, thermoplastics, and emulsions. Both the implant unit and the external device comprise a motion sensing unit, wherein the motion sensing units are different form each other, i.e. the motion sensing unit of the internal unit is a strain gauge and the motion sensing unit of the external device is an accelerometer.

According to another example not covered by the present invention, a use of the method disclosed herein for treatment of obstructive sleep apnea is presented. The treatment of obstructive sleep apnea, for which the method can be used, may for example comprise the following steps:
- receiving a modulation signal at an implant unit implanted at an internal location on an underside of a subject's chin (e.g.in the vicinity of the genioglossus muscle);
- applying the modulation signal to at least one pair of electrodes associated with the implant unit to generate an electric field;
- and causing modulation of a hypoglossal nerve or of an ansa cervicalis of the subject in response to the electric field generated by the at least one pair of electrodes, wherein the modulation of the hypoglossal nerve may be confined to a medial branch of the hypoglossal nerve and may be initiated from a single modulation site along the medial branch.

Therefore, the at least one pair of modulation electrodes may be configured for implantation through derma on the underside of the subject's chin and for location proximate to terminal fibers of the medial branch of the subject's hypoglossal nerve. In addition, the implantable unit and the electrodes may be configured to cooperate in order to generate an electric field adapted to modulate one or more of the terminal fibers of the medial branch of the hypoglossal nerve.

### Brief description of the drawings

The accompanying drawings, which are incorporated in and constitute a part of this specification, illustrate several examples of the disclosed subject matter. The drawings show the following:
- Fig. 1: shows a schematic depiction of the system disclosed herein according to a first embodiment;
- Fig. 2: shows a schematic depiction of a system according to a second embodiment;
- Fig. 3: shows a schematic depiction of a system according to a third embodiment.

### Detailed description of the drawings

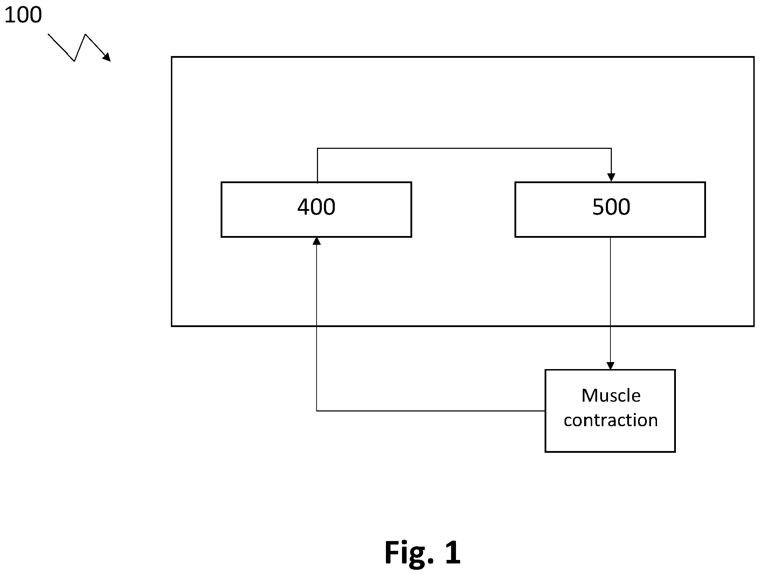

Fig. 1 shows a schematic depiction of a system 100 for measuring a degree of airway opening and/or airway obstruction, e.g. of muscle displacement of a subject in response to electrical nerve stimulation, wherein said muscle is part of the group of muscles responsible for the subject's breathing, e.g. the tongue, the genioglossus muscle or one or more of the infrahyoid strap muscles. The flow diagram of Fig. 1 may serve as representation of the underlying concept of the method. According to Fig. 1, a neuro stimulation system 100 configured for implementing the method comprises a control unit 500 and a motion sensing unit 400. Furthermore, the system 100 comprises implant unit 200 (not shown) configured for implantation in the proximity the genioglossus muscle, and an external device 300 (not shown) configured for electrical communication with the implant unit 200.

The implant unit 200 of the system 100 with which the method may be carried out, is implanted in a vicinity to the genioglossus muscle, which is innervated by the hypoglossal nerve, or to one of the infrahyoid muscles, which are innervated by the ansa cervicalis. The implant unit 200 may further comprise a transmitting element, which may comprise a secondary antenna or a coil, and an electric circuit which receives an electrical stimulation pattern through the electrical communication between the external device 300 and the implant unit 200. Upon receiving the electrical stimulation pattern, the electric circuit may generate an electric field via at the at least one pair of electrodes associated with the implant unit 200. If the electric field is strong enough and/or close enough to the nerve, the nerve will be stimulated and innervate the associated muscle, which will in turn contract. The specifications of the implant unit 200 are not limited to a certain embodiment as long as the implant unit 200 is configured for use with the method described herein.

The external device 300 of the system 100 with which the method may be carried out, is configured for location external to a patient and for placement underneath the subject's chin. The external device 300 may therefore be configured for affixation to the patient, for example, by adhering to the skin of the patient, or through a band or other device configured to hold the external device in place. The external device 300 may preferably comprise a housing, wherein the housing can be any suitable container configured for retaining electrical components. In addition, the housing may be any suitable size and/or shape and may be rigid or flexible. Examples of housings for the external device 300 may include one or more of patches, buttons, or other receptacles having varying shapes and dimensions and constructed of any suitable material. The specifications of the external device 300 are not limited to a certain embodiment as long as the implant unit 200 is configured for use with the method described herein.

In order to determine a movement (i.e. a contraction) of one or more muscles related to the subject's airway in response to the electric field applied by the implant unit 200, both the implant unit and the external device comprise a motion sensing unit, wherein the motion sensing unit of the implant unit is a strain gauge and the motion sensing unit of the external device is an accelerometer.

Lastly, the system 100 according to Fig. 1 comprises a control unit 500, for example as part of the external device 300. The control unit 500 may transmit modulation or stimulation input signals, e.g. the stimulation parameters, and power to the implant unit 200.

According to the method depicted in Fig. 1, the control unit 500 may also receive data corresponding to the movement of the one or more muscles related to the subject's breathing or airway determined by the motion sensing unit 400, and assigns to it a value relating to a degree of airway opening and/or airway obstruction. According to a preferred embodiment of the method, the control unit 500 is also able to adjust at least one of the stimulation parameters in response to the value indicative of the degree of airway opening and/or airway obstruction. Accordingly, the control unit 500 may comprise one or more processors, which may include any electric circuit that configured to perform a logic operation on at least one input variable. The at least one processor of the control unit 500 may for example include one or more integrated circuits, microchips, microcontrollers, and microprocessors, which may be all or part of a central processing unit (CPU), a digital signal processor (DSP), a field programmable gate array (FPGA), or any other circuit known to those skilled in the art that may be suitable for executing instructions or performing logic operations. This way, a method for feedback-based or closed loop neuro stimulation is provided, wherein the efficacy of an ongoing stimulation session is determined by measuring the degree of airway opening and then automatically adjusting the stimulation parameters in response to the measured degree of airway opening. The degree of protrusion of the subject's tongue can for example be determined by detecting movement of the muscle associated with the tongue or by detecting movement of the subject's chin. Automatic adjustment may comprise the control unit 500 receives a value indicative of tongue movement from the motion sensing unit 400 and thereupon adjusts at least one stimulation parameter, either after a set time delay or more or less instantly (i.e. in real-time).

In accordance with Fig. 1, the control unit 500 can be part of the external device 300, of the implant unit 200, or of both the external device 300 and the implant 200.

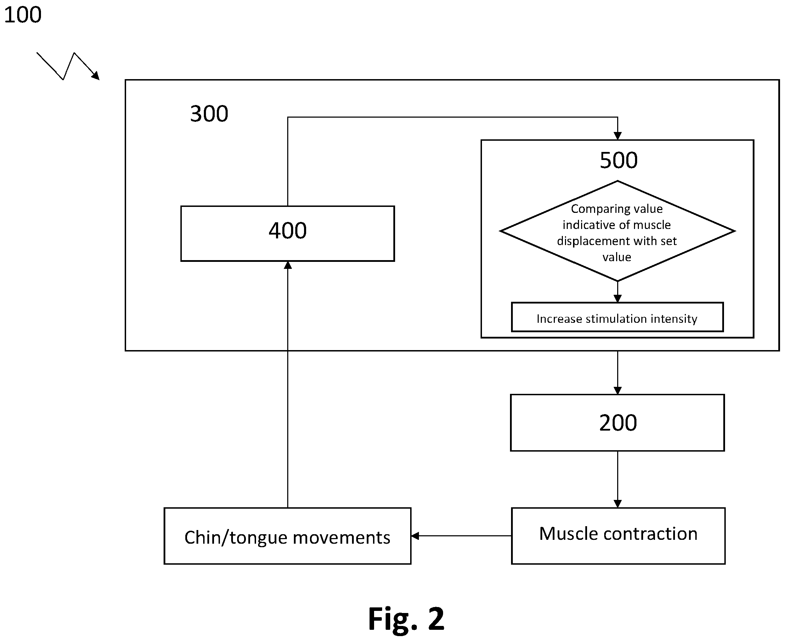

Fig. 2 shows a schematic depiction of a system 100 according to a second embodiment for measuring a degree of airway opening and/or airway obstruction, e.g. muscle displacement of a subject in response to electrical nerve stimulation. In comparison to the system of Fig. 1, the system 100 of Fig. 2 is further specified in that both the motion sensing unit 400 and the control unit 500 are shown as parts of the external device 300. In this case, the motion sensing unit 400 may preferably comprise at least one inertial measuring unit, e.g. an accelerometer.

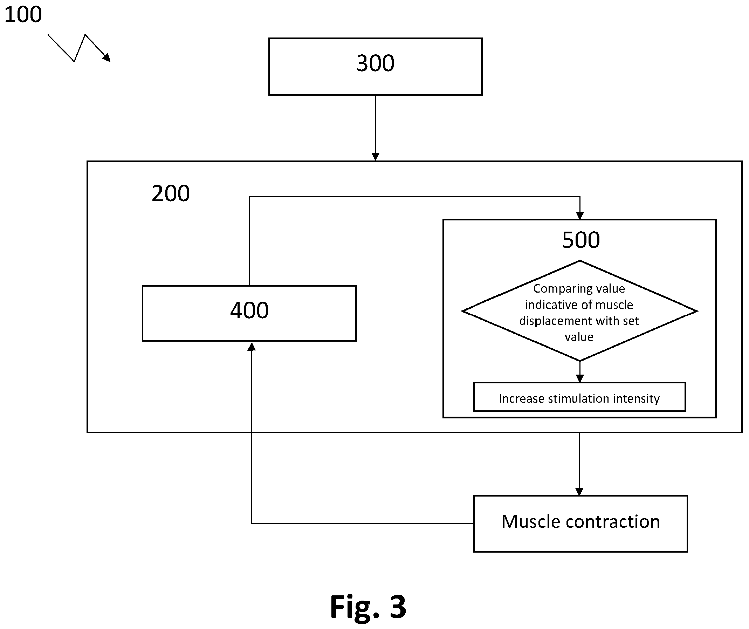

Fig. 3 depicts a flow diagram of a third embodiment of a system 100 for measuring a degree of airway opening, e.g. displacement of a subject in response to electrical nerve stimulation. The system 100 of Fig. 3 is further specified in that both the motion sensing unit 400 and the control unit 500 are shown as parts of the implant unit 200. In this case, the motion sensing unit 400 may preferably comprise at least one strain unit configured for detecting muscle contraction.

The invention is not limited to one of the embodiments described herein but may be modified in numerous other ways.

All features disclosed by the claims, the specification and the figures, as well as all advantages, including constructive particulars, spatial arrangements and methodological steps, can be essential to the invention either on their own or by various combinations with each other.

### List of reference numerals

- 100: System
- 200: Implant unit
- 300: External Device
- 400: Motion sensing unit
- 500: Control unit

## Claims

1. A system Z (100) for implementing a method of measuring a degree of muscle contraction of a subject in response to electrical nerve stimulation, the system comprising:
- an implant unit (200) configured for implantation in proximity to the genioglossus muscle;
- an external device (300) configured for location external to the subject and for placement underneath the subject's chin and configured for communication with the implant unit;
wherein both, the implant unit and the external device, comprise a motion sensing unit (400),
wherein the motion sensing unit of the implant unit is a strain gauge and the motion sensing unit of the external device is an accelerometer,
- a control unit (500) in electrical commutation with both of the motion sensing units
wherein the external device (300) is further configured for generating a modulation signal, the modulation signal comprising an electrical stimulation pattern;
wherein the implant unit (200) is further configured for applying the modulation signal to at least one pair of electrodes associated with the implant unit implanted inside the subject's body;
wherein the motion sensing units (400) are configured for detecting movement of one or more muscles related to the subject's airway in response to the modulation signal applied to the at least one pair of electrodes;
wherein the control unit (500) is configured for assigning to the determined movement a value that is indicative of the degree of airway opening and/or of airway obstruction.

## Patentansprüche

1. System (100) zum Implementieren eines Verfahrens zum Messen eines Muskelkontraktionsgrads eines Subjekts in Reaktion auf elektrische Nervenstimulation, wobei das System umfasst:
- eine Implantateinheit (200), die zur Implantation in der Nähe des Musculus genioglossus ausgelegt ist;
- eine externe Vorrichtung (300), die zur Positionierung außerhalb des Subjekts und zur Platzierung unter dem Kinn des Subjekts ausgelegt und zur Kommunikation mit der Implantateinheit ausgelegt ist;
wobei sowohl die Implantateinheit als auch die externe Vorrichtung eine Bewegungsabfühleinheit (400) umfassen,
wobei die Bewegungsabfühleinheit der Implantateinheit ein Dehnungsmessstreifen ist, und die Bewegungsabfühleinheit der externen Vorrichtung ein Beschleunigungsmesser ist,
- eine Steuereinheit (500) in elektrischer Kommutation mit beiden Bewegungsabfühleinheiten;
wobei die externe Vorrichtung (300) ferner zum Generieren eines Modulationssignals ausgelegt ist, wobei das Modulationssignal ein elektrisches Stimulationsmuster umfasst;
wobei die Implantateinheit (200) ferner zum Anlegen des Modulationssignals an mindestens ein Paar von Elektroden ausgelegt ist, das mit der Implantateinheit assoziiert ist, die innerhalb des Körpers des Subjekts implantiert ist;
wobei die Bewegungsabfühleinheiten (400) dazu ausgelegt sind, Bewegung von einem oder mehreren Muskeln, die mit dem Atemweg des Subjekts in Beziehung stehen, in Reaktion auf das Modulationssignal, das an das mindestens eine Paar von Elektroden angelegt wird, zu detektieren;
wobei die Steuereinheit (500) dazu ausgelegt ist, der bestimmten Bewegung einen Wert zuzuweisen, der den Grad der Atemwegsöffnung und/oder der Atemwegsbehinderung angibt.

## Revendications

1. Système (100) pour la mise en œuvre d'un procédé de mesure d'un degré de contraction musculaire d'un sujet en réponse à une stimulation nerveuse électrique, le système comprenant :
- une unité d'implant (200) configurée pour être implantée à proximité du muscle génioglosse ;
- un dispositif externe (300) configuré pour un emplacement externe au sujet et pour une mise en place sous le menton du sujet et configuré pour communiquer avec l'unité d'implant ;
l'unité d'implant et le dispositif externe comprenant tous deux une unité de détection de mouvement (400),
l'unité de détection de mouvement de l'unité d'implant étant une jauge de contrainte et l'unité de détection de mouvement du dispositif externe étant un accéléromètre,
- une unité de commande (500) en commutation électrique avec les deux unités de détection de mouvement,
le dispositif externe (300) étant en outre configuré pour générer un signal de modulation, le signal de modulation comprenant un motif de stimulation électrique ; l'unité d'implant (200) étant en outre configurée pour appliquer le signal de modulation à au moins une paire d'électrodes associées à l'unité d'implant implantée à l'intérieur du corps du sujet ;
les unités de détection de mouvement (400) étant configurées pour détecter un mouvement d'un ou plusieurs muscles liés aux voies respiratoires du sujet en réponse au signal de modulation appliqué à l'au moins une paire d'électrodes ; l'unité de commande (500) étant configurée pour affecter au déplacement déterminé une valeur indicative du degré d'ouverture des voies respiratoires et/ou d'obstruction des voies respiratoires.
